Europäisches Patentamt

European Patent Office

Office européen des brevets

(19)

(11) Publication number: **0 138 150**
**A2**

(12) **EUROPEAN PATENT APPLICATION**

(21) Application number: **84111795.5**

(22) Date of filing: **03.10.84**

(51) Int. Cl.⁴: **C 12 M 1/40**

(30) Priority: **05.10.83 US 539253**

(43) Date of publication of application: **24.04.85**
**Bulletin 85/17**

(84) Designated Contracting States: **BE DE FR GB IT LU NL**

(71) Applicant: **E.I. DU PONT DE NEMOURS AND COMPANY,**
**1007 Market Street, Wilmington Delaware 19898 (US)**

(72) Inventor: **Pace, Salvatore Joseph, 2208 Dorval Road,**
**Wilmington Delaware 19810 (US)**

(74) Representative: **von Kreisler, Alek, Dipl.-Chem. et al,**
**Deichmannhaus am Hauptbahnhof, D-5000 Köln 1 (DE)**

(54) **Diagnostic electrode membranes utilizing specific enzyme/ionophore pairs.**

(57) A hydrophobic, chemically inert, dielectric polymer membrane for the potentiometric detection of analytes is disclosed. The membrane has bonded to one surface an enzyme capable of catalyzing the hydrolysis of the analyte to yield an ion. The interior of the membrane contains an ionophore which is capable of complexing with the ion.

UREA NITROGEN SLOPE RESPONSE

TITLE                    IP-0365

DIAGNOSTIC ELECTRODE MEMBRANES
UTILIZING SPECIFIC ENZYME/IONOPHORE PAIRS

## TECHNICAL FIELD

This invention relates to an electrode membrane useful in determining the concentration of a specific constituent (analyte) in a body fluid or other liquid test sample. This invention also relates to electrode half cells making use of this membrane as well as methods for the determination of analyte concentrations in liquid test samples by means of these half cells.

## BACKGROUND ART

Guilbault and Montalvo describe a urea electrode comprising a Beckman cationic glass electrode coated with a 60-350μm thick layer of acrylamide having urease physically immobilized therein. The urease catalyzes the conversion of urea to ammonium ion ($NH_4^+$) and carbon dioxide ($CO_2$). The ammonium ion so generated reacts with the glass electrode to produce an observable change in potential. [J. Amer. Chem. Soc., Volume 91, 2164 (1969)]

These same workers describe the use of a nylon mesh as a support for the enzyme-polymer layer and the use of cellophane as a cover for the enzyme-polymer layer. The cellophane covered electrode was shown to have increased stability compared to the uncovered electrode. [Analytical Letters, Volume 2, 283 (1969)]

The electrodes described by these workers are subject to serious limitations which render them disadvantageous for certain clinical applications. [J. Amer. Chem. Soc., Volume 92, 2534 (1970)] In particular, the electrode responses are affected by ions other than $NH_4^+$, notably sodium ($Na^+$) and potassium ($K^+$) ions. These same workers found that $Na^+$ ion concentration must be less than one-half the urea concentration, and the $K^+$ ion concentration must be less than one-fifth the urea concentration to obtain electrode responses which are substantially independent of these ions. These workers speculate that these restraints on $Na^+$ ion and $K^+$ ion are such that adequate buffering capacity could not be obtained with traditional phosphate salt buffers and that buffers such as Tris would be required. Tris buffers, in turn, could pose serious limitations on the use of such electrodes for analytical purposes.

In addition, the stability and the response times (typically 25 to 60 seconds to reach 98% of the steady state value) of these electrodes were found to be a function of the thickness of the enzyme-polymer membrane. Because of the inherent difficulty and expense of manufacturing such electrodes with a high degree of uniformity, it would be necessary to calibrate individually each electrode before making a measurement of clinical importance. These electrodes suffer from a further disadvantage in that even with a cellophane coating which retards leaching of enzyme from the polymer membrane, these electrodes begin to lose activity after three weeks.

Tran-Minh et al. describe a urea sensitive electrode comprising a Beckman cation-sensitive glass electrode dip coated with a membrane consisting of bovine serum albumin (BSA), glutaraldehyde and urease. [C. R. Acad. Sc. Paris, Ser. C, Volume 275, 309 (1972)] When the urease contacts urea in the test solution, $NH_4^+$ is generated. The $NH_4^+$ interacts with the cation-sensitive glass electrode to produce a change in potential. Although the electrode is sensitive to $NH_4^+$, it is affected by the presence of monovalent cations other than $NH_4^+$, notably $K^+$. Because

0138150

of the interference by cations other than that generated by the enzyme, these workers tested the electrode in solutions using Tris buffer rather than a $Na^+$ or $K^+$ based buffer.

Finally, Guilbault and Nagy describe an improved urea electrode using an ammonium ion sensitive membrane comprising silicone rubber having the antibiotic nonactin dispersed therein. These membranes were placed on the end of a glass tube filled with 0.1 N $NH_4Cl$ and in which silver tape was used as an inner reference electrode. The active surface of the ammonium ion sensitive electrode was covered with a polymer membrane having urease physically immobilized therein. [Analytical Chemistry, Volume 45, 417 (1973)] This bi-layered electrode showed improved immunity to interference by $K^+$ ion. However, these electrodes still suffered from prolonged response times, typically 60 to 180 seconds depending on the urea concentration and the thickness of the enzymatically active polymer layer. These electrodes would be difficult to manufacture with a high degree of uniformity. Therefore, individual calibration would be required before making measurements of clinical importance. In addition, enzyme leaching would cause the electrode to lose activity with the passage of time. Because such an electrode would be expensive to fabricate, it would be wasteful to discard one after a single use. If one electrode were to be used for periodic measurements, re-calibration would be necessary before each use because of the leaching problem. Finally, these electrodes, like all the electrodes described above, require a substantial volume of test sample to operate effectively.

There is a need for a membrane suitable for the construction of uniform, inexpensive, disposable, sta-

0138150

ble, responsive electrode half cells which can selectively detect non-ionic analytes in small volumes of body fluids or other liquid test samples.

## DISCLOSURE OF THE INVENTION

The electrode membrane of this invention comprises a hydrophobic, chemically inert, dielectric polymer having an ionophore dispersed therein and an enzyme bound to a surface thereof, wherein the enzyme is capable of reacting with a substrate of interest to produce an ion for which the ionophore has specific affinity. In a preferred embodiment, the enzyme is covalently bound to the electrode membrane.

## BRIEF DESCRIPTION OF THE DRAWINGS

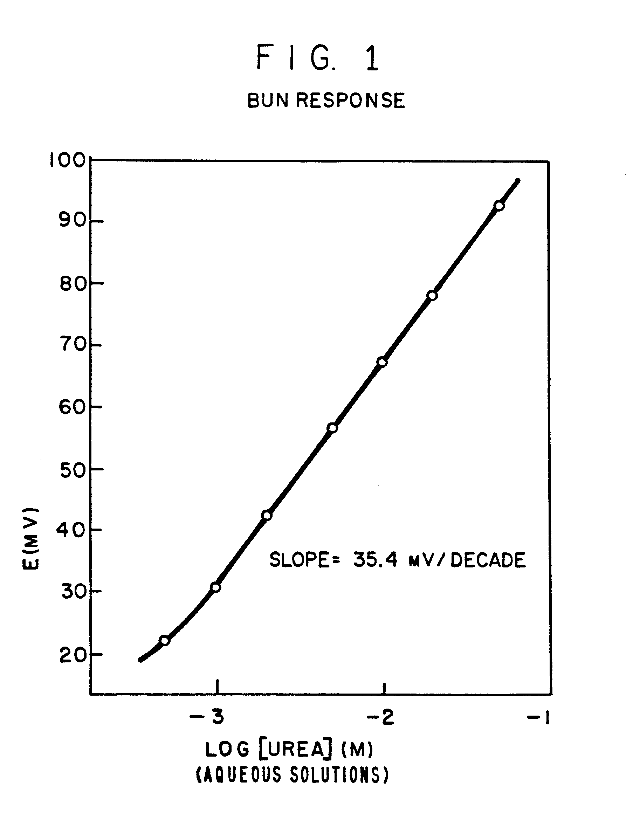

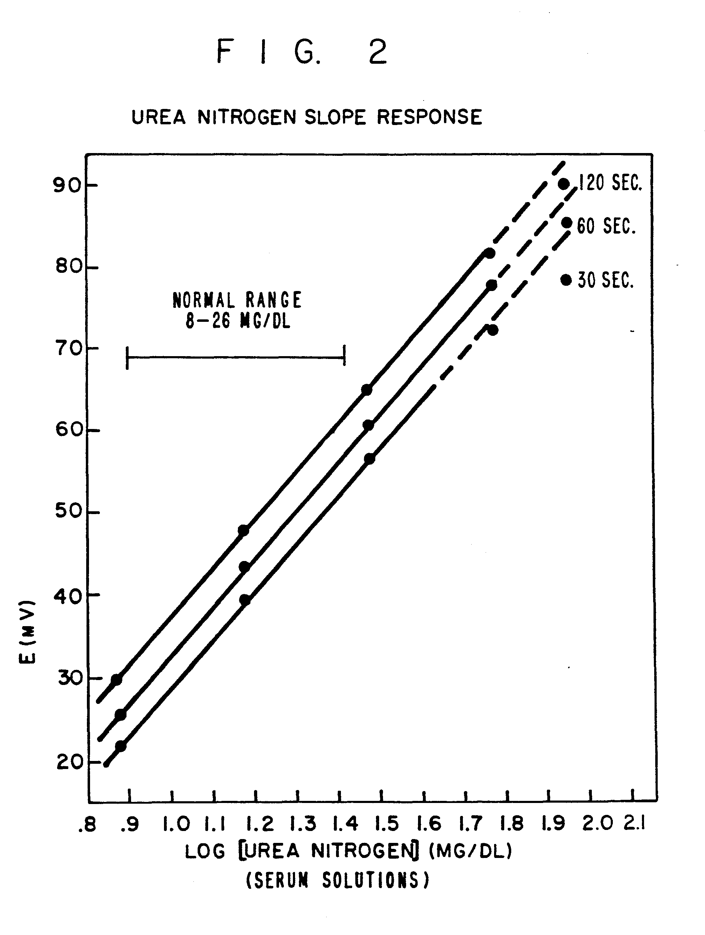

The drawings consist of two figures: Figure 1 is a graph showing measured potential vs. Log [Urea] in aqueous solution and Figure 2 is a graph showing measured potential vs. Log [Urea Nitrogen] in serum solutions.

## DETAILED DESCRIPTION

The membrane of this invention achieves its specificity for the analyte of interest by coupling together two highly selective chemical reactions and localizing these chemical reactions at a single interface, the membrane/test-solution interface. An enzyme is localized at the test-solution side of the interface where it specifically catalyzes the breakdown of analyte leading to the production of an ion. The ion so produced is selectively bound by an ionophore localized within the membrane. Hence, two highly selective reactions occur on either side of the interface, one is the enzymatic production of an ion, and the other is the specific complexation of the ion with an ionophore which is preselected for its affinity for the ion.

The complexation reaction results in a chemical equilibrium among ion, free ionophore, and complex. This complexation reaction is localized within a zone extending about 50Å into the interior of the membrane because the ionophore is insoluble in aqueous solution and the ion is not significantly soluble in the membrane unless it is complexed by the ionophore whose migration into the membrane interior is limited by the high viscosity of the membrane and the relatively large size of the ion/ionophore complex. Hence, no significant ion depletion occurs within the time frame of the measurement (i.e., minutes).

The entire enzymatic action occurs within a zone extending about 1000Å from the interface into the test-solution.

The overall response of a half cell using the membrane of this invention is controlled by the rate of mass transport of substrate (analyte) to the enzyme layer on the membrane surface, the rate of enzyme catalysis, and the rate of ion diffusion away from the interface. By design, the mass transport is the rate limiting step so that the half cell is insensitive to enzyme activity variations.

A steady state signal may be measured which reflects a rate of ion generation equal to the rate of ion diffusion away from the membrane surface. Alternatively, the rate of potential change may be measured.

A charge gradient is established on each side of the interface. The charge gradient that is established at the membrane side of the interface is compact. An unbound counter-ion on the solution side of the interface readily diffuses in the low viscosity aqueous test solution, resulting in a diffuse gradient of charge on the solution side. Charge neutrality is maintained by the counter-ion which does not play a significant role in the generation of an electrical potential.

The polymer from which the electrode membrane can be fabricated must be hydrophobic, chemically inert and dielectric, having a dielectric constant typically about 4.55. Various polymers are suitable; these include at least the following:

Poly(vinyl chloride) [PVC]

Cellulose Acetate

Poly(bisphenol-A carbonate)

Polysiloxane/Poly(bisphenol-A carbonate)
    block copolymer

Poly(methylmethacrylate)

Poly(vinylidene chloride)

Polystyrene

Lower alkyl acrylate and methacrylate
    polymers and copolymers

Polyurethane

Silicone rubber

Of the foregoing listed polymers, carboxylated PVC is preferred.

Generally, the polymer is combined with a plasticizer to provide a certain amount of polymer swelling. This is necessary to allow for mobility of ionophore in the membrane.

Suitable plasticizers include dioctyl adipate, tris(2-ethyl-hexyl) phosphate, dibutyl sebacate, o-nitrophenyl octyl ether, diphenyl ether, dinonyl phthalate, dipentyl phthalate, di-2-nitrophenyl ether, glycerol triacetate, tributyl phosphate, and dioctyl phenyl phosphate.

The membranes of this invention are usually made by forming a solution of the ionophore, polymer and plasticizer (if one is used) in a volatile organic solvent, screen printing or casting the solution onto the desired surface or into the desired shape and then removing the solvent by evaporation. The enzyme is then bound to the polymer surface as described below.

The ionophore which is to be dispersed within the membrane and the enzyme which is to be bound to the membrane, either physically or chemically, must be chosen to form a chemically complementary pair, i.e. the enzyme must react with a substrate (the analyte of interest) to produce an ionic product for which the ionophore has specific affinity. For example, if the analyte is urea, the enzyme will be urease which reacts with urea to produce $NH_4^+$. The ionophore will be nonactin because of its specific affinity for $NH_4^+$. Examples of analyte, immobilized enzyme, ionic species monitored, and ionophore are displayed in Table 1 below:

0138150

## Table 1

| Analyte | Immobilized Enbyme | Ionic Species Monitored | Inophore |
|---------|--------------------|-----------------------|----------|
| BUN | Urease | $NH_4^+$ | Nonactin |
| Creatinine | Creatininase | $NH_4^+$ | Nonactin |
| Triglycerides | Lipase | $H^+$ | Trioctylamine |
| Phenylalanine | Decarboxylase | $HCO_3^-$ | P-octodecycloxy-m-chlorophenyl-hydrazone-mesoxalo-nitrile |
| Tyrosine | Decarboxylase | $HCO_3^-$ | P-ocotodecyloxy-m-chlorophenyl-hydrazonemesoxalo-nitrile |
| Asparagine | Asparaginase | $NH_4^+$ | Nonactin |
| Glutamine | Glutominase | $NH_4^+$ | Nonactin |
| Pencillin | -lactamase | $H^+$ | Trioctylamine |

The concentration of ionophore in the membrane is not critical. Typical concentrations are from about 0.5% to about 3.0% (ionophore weight to total membrane weight).

The preferred method of enzyme immobilization is covalent attachment of the enzyme to a glutaraldehyde modified surface of the membrane. The resulting configuration possesses the desirable characteristics discussed above. In general, the membrane surface of the polymer lacks suitable reactive groups for covalent attachment. Therefore, the surface must be derivatized, i.e., a reactive group must be attached or exposed and then reacted with a coupling agent which serves to link the membrane surface to the enzyme. Table 2 below lists suitable reactive groups, coupling agents, reactive groups on enzymes, and resulting linkage after coupling. The preferred polymer for making the membrane of this invention is PVC. The preferred reactive group is COOH. Carboxylated PVC is commercially available or can be made from PVC by using the method of copolymerization of PVC with maleic anhydride, J. Polysci. Vol. 5, p. 253 (1950). The preferred coupling agent is glutaraldehyde, and the resulting linkage between the glutaraldehyde and the enzyme after coupling will be an amide.

## Table 2

| Reactive Group on Membrane Surface | Intermediate "Coupling agent" | Reactive Group on Reagent | Coupling Linkage Type |
|---|---|---|---|
| -COOH | $\overset{O}{\overset{\|}{H-C}}-(CH_2)_5-\overset{O}{\overset{\|}{C}}-H$ | $H_2N-$ or $HOOC-$ | $-NH-\overset{O}{\overset{\|}{C}}-$ "amide" |
| $-R-NH_2$ | $CH_3-N=C-N-CH_3$ (Carbodiimide) | $HO-\overset{O}{\overset{\|}{C}}-$ | $-NH-\overset{O}{\overset{\|}{C}}-$ "amide" |
| (phenyl)$-NH_2$ | (phenyl)$-\overset{\oplus}{N_2}\overset{\ominus}{Cl}$ "diazonium" | $HO-$(phenyl)$-$ | (phenyl)$-N=H$ (cyclohexyl with HO) "diazo" |
| (phenyl)$-NH-NH_2$ | | $-\overset{O}{\overset{\|}{C}}-$ | (phenyl)$-NH-M=C$ "hydrazide" |
| -SH | | HS- | -S-S- "disulfide" |
| "maleic anhydride" | | $H_2N-$ | $\overset{COOH}{...}\overset{CONH-}{...}$ "amide" |
| -COOH | $-\overset{O}{\overset{\|}{C}}-CH_3$ | $H_2N-$ | -CONH- "amide" |
| -CHO | Shiff. Base | $H_2N-$ | -C=N- |
| -SH | $CH_3-N=C=N-CH_3$ | HOOC- | -S-CO- "Thioamide" |
| (OH,OH diol) | CNBr | $H_2N-$ | (OCO-NH-, OH) |
| (triazine: Cl, CH_3) | Cyanuric Chloride | $H_2N-$ | (triazine: NH-, CH_3) |

The membrane of this invention can be used to make a solid state half cell for diagnostic applications. The half cell is described extensively in copending application S.N. 461,479, filed January 27, 1983. The half cell using the membrane of this invention is a multilayered device comprised of

(a) a dimensionally stable chemically inert electrically insulating substrate layer having coated thereon

(b) a terminated layer of electroconductive material, the conductivity of which is at least $1 \times 10^2$ $(ohms-cm)^{-1}$ having coated thereon

(c) a layer of finely divided particles of carbon uniformly dispersed in a matrix of organic polymeric binder having coated thereon

(d) the membrane of this invention, the membrane having an impedance at least two orders of magnitude greater than the impedance of the carbon layer, the interface between the carbon dispersion layer and the membrane of this invention comprising a zone in which the layers are partially interdiffused, and in use, layers (b) and (c) being shielded from both chemical and electroconductive contact with any analyte which may be present.

As used herein, the term "half cell" refers to an assemblage of interfaces at which ionic motion is converted to electron motion. A complete sensor is comprised of two such half cells which may also be referred to as electrodes, one of which is an indicator and the other a reference half cell.

The dispersed carbon layer consists essentially of a dispersion of finely divided particles of carbon in a matrix of dielectric organic hydrophobic polymer. The precise configuration of the carbon particles is not critical. For example, carbon black as

11

**0138150**

well as finely divided pellets and charcoal particles can be used. However, it is essential that the amount of carbon be sufficient that the carbon polymer dispersion layer has an electrical impedance of less than about 1% of the impedance of the ion-selective membrane.

However, in view of the fact that the dispersed carbon layer must be partially diffused into the ion-selective membrane, it will be apparent that the carbon particles must be finely divided; that is, they must be substantially smaller than the thickness of either the membrane layer or the carbon layer. Depending on the thickness of the layer, which is not by itself critical, a particle size of from 1 to 20 μm is preferred, from 1 to 10 μm being particularly preferred.

The particular organic polymer used as matrix for the carbon particles is, likewise, not particularly critical so long as the polymer is a dielectric and forms a matrix which is nonreactive with both the carbon and the ion-selective membrane layer. For example, all of the polymers which are suitable for the matrix of the membrane layer are also suitable as the matrix for the dispersed carbon layer. In addition, more highly crystalline polymers such as polyethylene and polypropylene can be used.

Suitable matrix polymers for the dispersed carbon layer also include film-forming oleophilic polymers such as poly(caprolactone); polyacrylate and alpha-alkyl polyacrylate esters, e.g., polymethacrylate, polymethyl methacrylate and polyethyl methacrylate; vinylidene chloride copolymers, e.g., vinylidene chloride/acrylonitrile, vinylidene chloride/methacrylate and vinylidene chloride/vinylacetate copolymers, ethylene/vinyl acetate copolymers; polyethylene; polyvinyl esters, e.g., polyvinyl acetate/acrylate, polyvinyl acetate/methacrylate and polyvinyl acetate; copolyesters, e.g., those prepared from the reaction product of a polymethylene glycol of the formula $HO(CH_2)_nOH$, wherein n is a whole number 2 to 10 inclusive, and (1) hexahydroterephthalic, sebacic and terephthalic acids, (2) terephthalic, isophthalic and sebacic acids, (3) terephthalic and sebacic acids, (4) terephthalic and isophthalic acids, and (5) mixtures of copolyesters prepared from said glycols and (i) terephthalic, isophthalic, sebacic and adipic acids; nylons or polyamides, e.g., N-methoxymethyl polyhexamethylene adipamide; synthetic rubbers, e.g., butadiene/acrylonitrile copolymers, and chloro-2-butadiene-1,3-polymers; block copolymers, e.g., polystyrene-polybutadiene-polystyrene and polystyrene-polyisoprene; poly(vinyl chloride) and copolymers, e.g., poly(vinyl chloride/acetate); polyvinyl acetal, e.g., poly(vinyl butyral), poly(vinyl formal) polyurethanes; polycarbonates; polystyrene; phenolic resins; and melamine-formaldehyde resins.

In some instances, plasticization of the matrix polymer for the dispersed carbon layer may not be needed. However, to minimize the formation of unwanted concentration gradients between the ion-selective membrane and the dispersed carbon layer, use of the same, or at least similar, polymers in the two layers is preferred. Thus, when PVC plasticized with dioctyl phthalate is used as the matrix for the membrane layer, a most convenient and economical way of minimizing migration of species between the layers is to use the same plasticized PVC composition as the matrix for the dispersed carbon layer. Furthermore, interdiffusion of the layers is facilitated by the mutual solubility of the matrices.

As will be made clear in the following discussion of the fabrication of the electrode half cells of the invention, it is preferred that the dispersed carbon layer matrix polymer be plasticized to some extent to facilitate interdiffusion of the membrane and carbon layers.

Generally, a wide variety of conductive materials can be used in the half cells so long as the interface with the dispersed carbon layer has a stable conductor contact potential difference and is free from oxidation. Though noble metals such as silver and gold are often preferred as the conductive material, because of their chemical inertness, base metals such as copper, nickel, iron, tin, cadmium and aluminum can also be used, because they are not subject to stringent oxidative conditions in the device of the invention.

In all cases, the conductive layer(s) of the half cell must be provided with terminations by which the electrode can be connected to an electrometer-amplifier to measure any potential difference between the half cells. These terminations may merely be an extension of the conductive layer or they may be made by specially printing a contiguous conductive layer of either the same or a different conductive metal. The precise method of termination is not critical and will largely be determined by fabrication economics for any given performance level.

It is, of course, necessary that the conductor pattern which is not in contact with the dispersed carbon layer be shielded from test sample by a layer which is both electrically insulating and chemically inert with respect to the test sample. This is normally done by covering the nonchemically functional portion of the conductor pattern with a layer of chemically inert insulating material such as a polymer or glass. The polymers which are suitable for the matrix of the membrane and carbon layers are also in general suitable as an insulator for those portions of the conductive layer which need to be shielded.

The electrode half cell and the sensors made therefrom have a further advantage in that they can be fabricated in very small sizes and as very thin layers. The half cell is essentially a simple multilayer laminate, i.e., it is a stack of thin layers or films. A particularly convenient method of forming the layers is to screen print them on whatever substrate is used. For example, in the case of the conductive material, a dispersion of finely divided particles of the conductive material in an inert organic medium is formed, screen-printed in the desired pattern onto the insulating substrate and fired to sinter the conductive material and form a "continuous" conductive layer.

On the other hand, finely divided particles of the conductive material can be dispersed in a photosensitive medium, coated on the insulating substrate, exposed through an appropriate phototool to harden the dispersion in the desired pattern, and developed to remove the unexposed dispersion.

Likewise, the successive carbon and ionophore/enzyme layers can be applied by the same techniques or others which will be well known by those skilled in the art.

Of primary importance, however, is the formation of the interdiffusion zone between the carbon and ionophore/enzyme layers. The interdiffusion zone must not extend completely through either the ionophore/enzyme membrane or the carbon layer. However, the zone must be extensive enough to diffuse any charge gradient developed by the passage of current as required by the electrometer-amplifier used for the measurement. The reason for this is that there must exist zones of substantially pure ionophore/enzyme layer and substantially pure conductive layer to effect the transition from electron flow to ion flow in support of the electrical current without altering the half-cell potential. The concentration gradient is developed at the sample solution/ionophore/enzyme layer interface and extends inwardly toward the interdiffusion zone and a substantially zero concentration gradient should reside at the carbon layer side of the zone. On the other hand, from the carbon layer side of the interdiffusion zone, the carbon layer provides a conductive path for electrons and also allows the transport of ions at the interface to support current flow by the electrometer-amplifier.

Though in theory these criteria might be met by a large number of separate stacked layers, each having appropriate changes in concentration of ionophore and carbon particles, this would be very onerous and very expensive. A much easier way to accomplish this is to plasticize both of the matrix polymers whereupon the laminated layers will autogenically interdiffuse due to mutual solubilization. In addition, interdiffusion of the layers, either with or without plasticization, can be effected by the application of pressure and/or by the application of heat for a time sufficient to effect the proper degree of interdiffusion.

However, by whatever method the interdiffusion zone is formed, it is essential that the interdiffusion zone have a high-to-low dielectric gradient from the membrane to the carbon layer. In addition, it is essential that the interdiffused zone be stable. For this last reason, it is very desirable to incorporate a cross-linking agent in the binder component of

0138150

either or both of the polymer matrices to effect both physical and chemical stabilization of both the membrane and carbon layers as well as the interposed interdiffusion layer.

The following procedure is typical of the way in which the electrode half cells of the invention and sensors made therefrom are fabricated in layers using screen-printing techniques.

The various layers are built up on a planar substrate which typically has dimensions 1" x 2" x 0.025" (2.5 x 5.0 x 0.06 cm). The resultant structure is commonly referred to as a chip. The substrate must be chemically inert with respect to analyte and the layers placed thereon. It must also be dimensionally stable and immiscible with respect to the analyte and/or overlying layers. The dimensions, however, are not critical and may be varied. The composition of the substrate is chosen on the basis of cost, ease of manufacture, and durability. Although for the purposes of this discussion ceramic is chosen as the substrate, other substrates, for example, Mylar® polyester film, can be employed.

The first or bottommost functional layer of the device is the conductor, usually silver. A silver paste is screened over a ceramic substrate using a 250 mesh screen and allowed to dry for 5 to 10 minutes at room temperature. It is then dried for an additional 10 minutes at 150°C and finally fired for 30 minutes at 850°C.

The second functional layer of the device is the insulator, usually glass, which is printed over the conductor pattern using a 165 mesh screen in such manner that the conductor paths are protected, but the contact area of the electrode is exposed. It is dried for approximately 5 minutes at room temperature and for an additional 10 minutes at 150°C.  Finally, it is fired for 30 minutes at 850°C.  If a polymeric substrate is used then the same function can be served by use of a dielectric photosensitive polymer system (e.g., a dry film photoresist) to form the appropriate protective pattern.

The third functional layer of the device, the carbon layer, is printed above the conductor pattern. A 165 mesh screen is employed.  The carbon layer is dried for approximately 5 minutes at room temperature and cured for another 20 minutes at 160°C.

The fourth layer of the device, the polymer/ionophore layer, is printed above the carbon pattern using a 165 mesh screen.  The composition of this layer is variable, depending on the species to be detected.

Finally, the enzyme is bound to the enzyme/ionophore surface.  A preferred method of binding consists of layering an aqueous solution of glutaraldehyde on the surface, incubating for roughly ten minutes at room temperature and then suctioning off the excess. Next, the enzyme is layered on the glutaraldehyde treated surface.

Sufficient enzyme must be immobilized on the surface to ensure a substrate independent enzyme catalyzed reaction rate that is much faster than the mass transport rate of analyte to the polymer/ionophore surface.  Thus, diffusion is the rate limiting (or slow) step in the overall rate of ion generation. In the Michaelis-Menten equation, this condition is described as the limit of a zero-order reaction where $K_m \ll [S]$ ($K_m$ is defined as $([ES]/[E]+[S])$ and $[E]$ = enzyme concentration, $[S]$ = substrate concentration, $[ES]$ = activated complex concentration).  Under such reaction conditions, ion formation is described by the equation:

$$[Ion] = \frac{k_2[E][V^*]}{\emptyset}$$

where $k_2$ is the rate of ES dissociation,

$V^*$ is the maximum, substrate independent, reaction rate and $\emptyset$ is the permeability of substrate through the sample and barrier layer.

To ensure complete conversion of substrate at a rate that is 100 times faster than $\emptyset$, it is necessary that 10 units (M/min) of enzyme activity be immobilized on the surface. These calculations assume a typical permeability of $H_2O$ through a hydrophilic medium (e.g., protein) and a substrate concentration in the clinical range for human blood. In practice, the enzyme activity typically deposited on the polymer/ionophore is approximately 100 units (or 10 μL of a 10,000 U/ml preparation) which is 1000 times more activity than required and well beyond the threshold of a mass transport limited rate.

Using the materials described above, it is not necessary to carry out any special procedures to form the interdiffusion zone at the interface of the ionophoric and carbon layers. The reason for this was that in both layers the same plasticizer and same polymer were used, which gives to the layers a substantial degree of mutual solubility which, without further manipulation, results in formation and stabilization of the zone within a very short time after fabrication. Because the interdiffusion zone can be formed so easily by this method, it is a preferred way of fabricating this segment of the electrode half cells of the invention. The extent and rate of interdiffusion can be adjusted by increasing or decreasing the mutual solubility of the layers, e.g., by changing the composition and/or concentration of the plasticizer in either or both layers.

In most instances it will be preferred that the layered electrode half cell be disposed on the same substrate as a reference half cell similar to the sensing half cell, but in which a nonenzymatic protein, generally an albumin such as BSA, substitutes for the enzyme. The sensing half cell and reference half cell will be in spaced apart relationship. The resulting whole cell is coated with a continuous barrier layer of a hydrophilic polymer which allows small molecules ($<500\overset{\circ}{A}$) to penetrate to the enzyme. Examples of such polymers are cellulose, polyacrylamide and Carboset (available from Du Pont). In this embodiment, the whole cell self-corrects for ionic effects not attributable to enzyme catalysis of the analyte, i.e., background ion concentration in the test sample. In a preferred embodiment, both the enzyme-containing half cell and the reference half cell are constructed on the same ceramic substrate layer and the optional barrier layer covers both half cells, thereby isolating them from large molecules as well as restricting diffusion of enzymatically generated ions from the enzymatically active half cell to the reference half cell. Because the enzymatic reaction is localized at the polymer/ionophore surface where ion detection occurs, the ion produced by the enzymatic reaction will not interfere with the reference half cell potential within the time frame of a typical measurement. This is because the ion will not diffuse sufficiently fast to affect the reference half cell potential even if the two half cells are only about 1 mm apart. Hence, differential potentiometry (same sample applied to sensing and reference half cells) may be practiced to remove all sources of interferences encountered in biological specimens.

0138150

The preferred whole cell is connected to an electrometer-amplifier to measure voltage change as a function of analyte concentration in the test sample. The ceramic chip containing both half cells can be immersed in the test solution of interest or an aliquot of test sample can be applied to the surface of the whole cell.

## EXAMPLE

### PART I

### Fabrication of $NH_4^+$-Sensing Element

An ammonium ion ($NH_4^+$)-sensing element was fabricated essentially as described in copending patent application S.N. 461,479 filed January 27, 1983, which is incorporated herein by reference. In particular, the sensor consisted of a 1" x 2" x 0.025" (2.5 x 5 x 0.06 cm) alumina chip upon which were printed two terminated silver conductor patterns. The entire uncoated area of the substrate chip was covered with a layer of glass to shield the sides of the conductor pattern from analyte. Correspondingly, the sides of the dispersed carbon layer were shielded by the overlying ionophoric layer. The entire element was covered with a layer of Silastic® polymer film except for one side of the ion-selective membrane which was left exposed to provide for limited ionic contact with the analyte.

The first or bottommost functional layer of the sensing element, the silver conductor, was screen printed over the substrate using a silver paste and a 250 mesh screen. It was air-dried for 5 to 10 minutes, then dried for an additional 10 minutes at 150°C, and finally fired for 30 minutes at 850°C.

The second functional layer of the device, the glass insulator, was printed over the conductor pattern using a 165 mesh screen, in such manner that the conductor paths were protected but the contact area of the electrode was exposed. The layer was air-dried for 5 minutes, then dried for an additional 10 minutes at 150°C, and finally fired for 30 minutes at 850°C.

The third functional layer, the carbon layer, consisted essentially of finely divided carbon in a vinyl chloride/vinyl acetate copolymer matrix containing butyl cellulose acetate. This was screen printed above the conductor pattern using a 165 mesh screen. This layer was air-dried for 5 minutes and then cured for 20 minutes at 160°C.

The fourth layer of the device, the ionophoric layer, consisted of 5 parts nonactin per hundred parts of a solution consisting of 30% carboxylated poly-(vinyl chloride) and 70% diocytladipate in cyclohexanone. This layer was printed above the carbon pattern using a 165 mesh screen. It was air-dried for 10 minutes and then cured for 30 minutes at 35°C.

A solution of 12.5% glutaraldehyde in 25 mM sodium phosphate buffer, pH 7.5, was then layered dropwise over the ionophoric layer in an amount sufficient to completely cover the surface. After a ten minute incubation at room temperature, excess solution was removed by suctioning, and the surface was blown dry with nitrogen. A solution of urease (7,000-10,000 U/mL, 100 mg/mL in 50 mM sodium phosphate buffer, pH 7.5) was then layered dropwise over the ionophoric layer in an amount sufficient to completely cover it. After a ten minute incubation at room temperature, excess enzyme solution was removed by suctioning and the surface was air-dried for 1 hour.

The reference half-cell was prepared exactly as described above except that albumin was coupled to the ionophoric layer rather than urease.

Both the reference and the sensing half-cells were then overlaid with Silastic® printed witn a 1b5 mesh screen. They were air-dried for 10 minutes and cured for 1 hour at 50°C in a humidified $CO_2$ atmosphere.

## PART 2

### Measurement of Urea

The electrodes fabricated in Part 1 above were used to generate a standard curve for urea using aqueous standards prepared by dissolving urea to the desired concentration in 50 mM sodium phosphate buffer, pH 6.8. Figure 1 shows the electrode response in millivolts (mV) as a function of the log molar urea concentration using a two minute measurement period. The response was linear over two orders of magnitude in concentration and had a slope sensitivity of 35.4 mV/decade change in urea concentration.

## PART 3

### Measurement of Blood Urea Nitrogen (BUN)

BUN was measured in human serum samples using the electrodes fabricated in Example 1 above. Prior to use, each electrode was calibrated with a 20 mM urea standard. Figure 2 shows the electrode response in mV as a function of the log BUN concentration in mg/dL for 3 different times. The table below shows the performance of the electrodes in terms of accuracy and precision at 3 different concentrations of BUN. The reference method was the aca$^{TM}$ discrete clinical analyzer BUN method.

### Performance Characteristics of the BUN-Sensing Electrode

| [BUN] mg/dL | ACCURACY (%) | PRECISION C.V. (%) |
|---|---|---|
| 15.7 | 100 | 7.6 |
| 37.1 | 99.7 | 9.2 |
| 58.5 | 100.3 | 10.0 |

0138150

CLAIMS:                                    IP-0365

1. An electrode membrane for the determination of an analyte in a liquid test sample, comprising a hydrophobic, chemically inert, dielectric polymer having bound to a surface thereof an enzyme capable of reacting with the analyte to produce an ion, and having dispersed therein an ionophore capable of complexing with the ion.

2. The membrane of Claim 1 wherein the polymer is selected from the group consisting of poly(vinyl chloride), cellulose acetate, poly(bisphenol-A carbonate), polysiloxane/poly(bisphenol-A carbonate) block copolymer, poly(methylmethacrylate), poly(vinylidene chloride), polystyrene, lower alkyl acrylate and methacrylate polymers and copolymers and polyurethane.

3. The membrane of Claim 2 wherein the polymer is poly(vinyl chloride).

4. The membrane of Claim 2 wherein the poly-(vinyl chloride) is carboxylated.

5. The membrane of Claim 1 wherein the enzyme is urease and the ionophore is nonactin.

6. The membrane of Claim 1 further comprising a plasticizer.

7. The membrane of Claim 5 wherein the plasticizer is dioctyl adipate.

8. The membrane of Claim 5 wherein the urease is bound to the membrane surface using a coupling agent.

9. The membrane of Claim 8 wherein the coupling agent is glutaraldehyde.

10. An electrode half-cell comprising

(a) a dimensionally stable, chemically inert, electrically insulating substrate layer having coated thereon

(b) a terminated layer of electroconductive material, the conductivity of which is at least 1 x $10^2$ $(ohms-cm)^{-1}$ having coated thereon

(c) a layer of finely divided particles of carbon uniformly dispersed in a matrix of organic polymeric binder having coated thereon

(d) a hydrophobic, chemically inert, dielectric polymer membrane having an ionophore dispersed therein, the membrane having an impedance at least two orders of magnitude greater than the impedance of the carbon layer, the interface between the carbon dispersion layer and the membrane comprising a zone in which the layers are partially interdiffused, the membrane having bound on its external surface,

(e) an enzyme capable of reacting with a nonionic analyte to produce an ion capable of complexing with the ionophore.

11. An electrode whole cell for the differential potentiometric measurement of a nonionic analyte in a test sample, comprising: a sensing half cell and a reference half cell, the sensing half cell being the half cell defined in Claim 10 and the reference half cell comprising

(a) a dimensionally stable, chemically inert, electrically insulating substrate layer having coated thereon

(b) a terminated layer of electroconductive material, the conductivity of which is at least 1 x $10^2$ $(ohms-cm)^{-1}$ having coated thereon

(c) a layer of finely divided particles of carbon uniformly dispersed in a matrix of organic polymeric binder having coated thereon

(d) a hydrophobic, chemically inert, dielectric polymer membrane having the ionophore dispersed therein, the membrane having bound to its external surface,

(e) a nonenzymatic protein.

0138150

12.   The whole cell of Claim 11 wherein the nonenzymatic protein is albumin.

13.   The whole cell of Claim 11 wherein the sensing half cell and the reference half cell are disposed on the same substrate layer in spaced apart relationship.

F I G.  1

BUN RESPONSE

LOG [UREA] (M)
(AQUEOUS SOLUTIONS)

SLOPE= 35.4 mV/DECADE

F I G.   2

UREA NITROGEN SLOPE RESPONSE

NORMAL RANGE
8-26 MG/DL